# EUROPEAN PATENT SPECIFICATION

(11) **EP 0 535 734 B1**
(45) Date of publication and mention of the grant of the patent: **02.06.1999**
(21) Application number: 92202940.0
(22) Date of filing: 24.09.1992
(51) Int. Cl.: A61K 31/71, A61K 47/44, A61K 9/00

(54) **Long acting injectable formulations containing hydrogenated castor oil**
Hydrierte Rizinusöl-enthaltende injizierbare Formulierungen mit verlängerter Wirkstoffabgabe
Formulations injectables à libération soutenue contenant de l'huile de ricine hydrogénée

(30) Priority: 30.09.1991 US 768797
(43) Date of publication of application: 07.04.1993
(73) Proprietor: Merck & Co., Inc., Rahway New Jersey 07065-0900 (US)
(72) Inventor: Chen, Elinor H., North Brunswick, NJ 08902 (US); Williams, James B., Freehold, NJ 07728 (US)
(74) Representative: Thompson, John Dr.

(56) References cited:
- EP-A- 0 413 538
- GB-A- 2 176 182

## Description

### BACKGROUND OF THE INVENTION

The avermectin series of compounds are potent anthelmintic and antiparasitic agents against internal and external parasites. The natural product avermectins are disclosed in US 4,310,519 to Albers-Schonberg et al., and the 22,23-dihydro avermectin compounds are disclosed in Chabala et al. US 4,199,569.

European Patent Publication No. 0 413 538 describes long acting injectable formulations consisting of triacetin and an avermectin compound.

British Patent Publication No. 2 176 182 describes macrolide antibiotics and *inter alia* injectable compositions containing them.

### SUMMARY OF THE INVENTION

This invention is concerned with the unexpectedly long duration of activity which is observed when avermectin injectable formulations are prepared using hydrogenated castor oil and hydrophobic or water immiscible carriers. Thus, it is an object of this invention to describe such a prolonged therapeutic effect. An additional object is to describe the avermectin compounds which may be employed in the long acting formulation. A still further object is to describe additional components which may be employed in the formulation. Additional objects will become apparent for a reading of the following description.

### DESCRIPTION OF THE INVENTION

This invention consists of an injectable formulation of a hydrogenated castor oil and an avermectin compound in a hydrophobic carrier which has been found to have a considerably prolonged duration of activity against internal and external parasites.

The hydrogenated castor oil imparts to the formulation an increased viscosity. While not wishing to be bound by theory, it appears that the increased viscosity together with the hydrophobicity of the carrier enable the injected formulation to remain at the injection site and form a "depot" of the active material which is slowly removed from the injection site over a prolonged period of time. The hydrogenated castor oil is readily prepared using normal techniques known to those skilled in the art of preparing hydrogenated castor oils and one suitable form of hydrogenated castor oil is available commercially under the trade name "Thixcin" from NL Industries.

The avermectin compounds have the following structure: 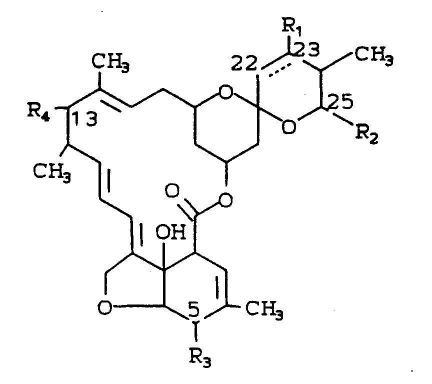 where the broken line indicates a single or a double bond at the 22,23-positions;
R₁ is hydrogen or hydroxy provided that R₁ is present only when the broken line indicates a single bond;
R₂ is alkyl of from 1 to 6 carbon atoms or alkenyl of from 3 to 6 carbon atoms or cycloalkyl of from 3 to 8 carbon atoms;
R₃ is hydroxy, methoxy or = NOR₅ where R₅ is hydrogen or lower alkyl; and
R₄ is hydrogen, hydroxy or 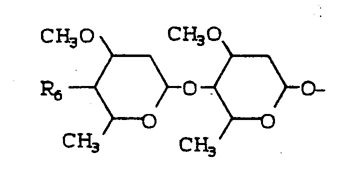
where R₆ is hydroxy, amino, mono-or di-loweralkyl amino or loweralkanoylamino.

The preferred compounds are avermectin Bla/Blb (abamectin), 22,23,dihydro avermectin Bla/Blb (ivermectin) and the 4''-acetylamino-5-ketoximino derivative of avermectin Bla/Blb. Both abamectin and ivermectin are approved as broad spectrum antiparasitic agents. The structures of abamectin and ivermectin are as follows: 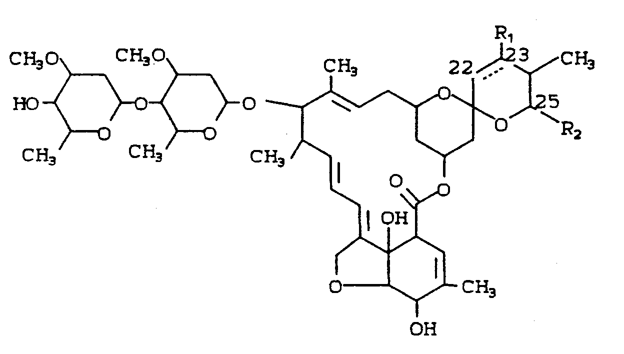 wherein for abamectin the broken line represents a double bond and R₁ is not present and for ivermectin the double bond represents a single bond and R₁ is hydrogen; and
R₂ is isopropyl or sec-butyl.

The 4''-acetyl amino-5-ketoximo derivatives of avermectin Bla/Blb has the following structural formula: 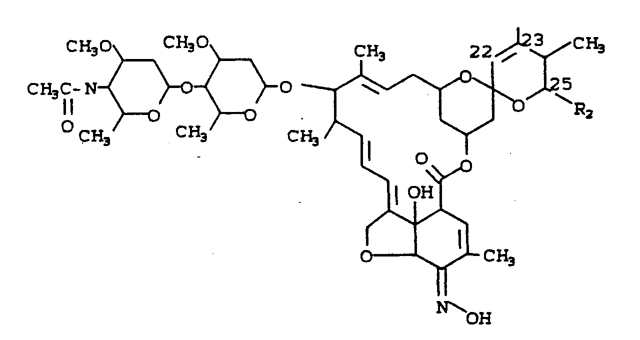 where R₂ is isopropyl or sec-butyl.

The avermectin products are generally prepared as a mixture of at least 80% of the compound where R₂ is sec-butyl and no more than 20% of the compound where R₂ is isopropyl.

The instant formulation is equally applicable to other compounds used for injection as long as such compounds are soluble in the mixture of the hydrogenated castor oil and hydrophobic carrier. Compounds that can be used in this formulation are, antiparasitic agents, antibiotics, therapeutic vitamin and mineral supplements, growth promoting agents, hormones, and other agents that are assisted in their therapeutic effect by having their effects extended over a prolonged period of time.

In the injectable forms of the avermectin formulation it has not been possible to provide a long acting formulation since the avermectins are insoluble in water and generally of low solubility in the oil carriers usually used for injection. Heretofore, injectable formulations of a maximum of 14 days have been possible. The current long acting formulations of avermectins provided for unacceptably large volumes of the injection fluid, or resulted in irritation at the injection site.

The instant formulations of an avermectin compound with hydrogenated castor oil in a hydrophobic carrier provides the advantages of a readily injectable formulation which provides the animal with treatment and protection against internal and external parasites for up to 42 days. The hydrogenated castor oil increases the viscosity of the formulation when present at from about 0.5 to 3% on a weight for volume basis. The material forms a depot and remains generally in the area of the injection site, however, it is still readily drawn into a syringe and expelled from the syringe into the tissue of the injection site, usually subcutaneously, without undue force being applied to the syringe. The increased viscosity and hydrophobicity of the injection material causes the material to be maintained as a depot to provide the necessary reservoir of active ingredient for a long acting effect. No pain or tissue damage has been observed at the site of injection either at the time of injection or during the 42 day course of therapy. In contrast to most oil based formulations, the formulation maintains a low viscosity even at high drug loads and even at temperatures as low as 5°C, a temperature at which most oil-based formulations become too viscous for practical use. Since previous injectable formulations only provided 14 days duration, the improved current formulation with 42 days duration avoids two additional injections which would be necessary to obtain 42 days protection with a 14 day duration injection and also avoids the stress of the extra handling necessary for the extra injections which increses illness and weight loss.

The hydrogenated castor oil formulation contains the avermectin compound in a hydrophobic physiologically acceptable injection solvent in which the avermectin compound is readily soluble. Any physiologically and pharmaceutically acceptable carrier may be used so long as the avermectin compound is soluble therein. Examples of such carriers are glyceryl triacetate (Triacetin), distilled acetylated monoglycerides (Myvacet™), miglyol™ 812, safflower seed oil and the like. Or a combination of such carriers. The formulations will generally be prepared to administer from 0.1 to 1 mg/kg preferably from 0.4 to 0.85 mg/kg and most preferably from about 0.6 to 0.7 mg/kg of the active ingredient. At a preferred dose volume of about 1 ml to treat 50 kg of animal body wight the formulation contains from 5 to 50 mg of avermectin compound per ml of solution or about 0.5 to 10% w/v preferably 2.5 to 5% w/v. However, depending upon the activity of the compound and the animal being treated, doses as low as 0.3% w/v of the avermectin compound are usable.

In addition to the hydrogenated castor oil, the avermectin and the hydrophobic carrier, the formulation can contain an antioxidant such as a propyl gallate, BHA (butylated hydroxy anisole), BHT, (butylated hydroxy toluene) monothioglycerol and the like. The antioxidants are generally added to the formulation at rates of from 0.01 to 2.0% (w/v).

Since the long acting formulation is intended for injection, it is necessary that it be sterilized. Heat sterilization is generally to be avoided since the avermectin compound would be unstable at autoclave temperatures. Rather, membrane sterilization is preferred. The sterile solution is then packaged aseptically.

The avermectin-hydrogenated castor oil long acting injectable formulation may be administered to warm blooded animals to provide up to 42 days of treatment and protection against external and internal parasites. Typically, the formulation is administered to domesticated animals such as cattle, sheep, pigs, dogs, horses, and the like.

The following example is provided in order that the invention might be more fully understood.

### EXAMPLE OF THE INVENTION

An ivermectin formulation for injection was prepared from the following formula:

| | |
|---|---|
| Ivermectin | 3.15% w/v |
| n-Propyl gallate | 0.02% w/v |
| Hydrogenated Castor Oil (Thixcin) | 1.5% w/v |
| Glyceryl triacetate (Triacetin) | qs 100% w/v |

Into a tared beaker 12.6 gm of ivermectin and 0.08 gm of n-propyl gallate are dissolved in 300 ml of glyceryl triacetate. The materials are filtration sterilized and placed in a dispersator/mixer, heated to about 50°C and the dispersator/mixer stirred at about 400 rpm. 6.0 gm of hydrogenated castor oil previously sterilized by gamma radiation is added into the warm solution with mixing. To the beaker is then added sufficient filtration sterilized glyceryl triacetate to prepare a 400 ml solution. A thickening of the solution is observed. The stirring rate is then increased to about 4000 rpm and the stirring is continued at 4000 rpm for about 25 ± 5 minutes. The stirring rate is reduced to about 400 rpm and the solution allowed to cool to ambient temperature. The solution is then packaged aseptically and stored at room temperature. No separation of the solution was observed. The viscosity of the solution was 500 +/- 100 cps as measured by a Brookfield viscometer with a spindle #2 at 25°C and 60 rpm.

The sterile viscous ivermectin solution was injected into calves at a dose of 630 mcg/kg at a dose volume of between 5 and 10 ml depending upon the weight of the animal. Randomly selected groups of animals were challenged with infection levels of Cooperia spp and Nematodirus helvetianus on days 28 and 42. The single injection of ivermectin was seen to be fully effective against the challenges to day 42.

## Claims

1. An injectable composition consisting of from 0.5 to 10% w/v of an avermectin compound in a pharmaceutically acceptable hydrophobic carrier and from 0.5 to 3% w/v of a hydrogenated castor oil.

2. The composition of Claim 1 wherein the avermectin compound is present at from 2.5 to 5% w/v of the formulation.

3. The composition of Claim 1 wherein the avermectin compound has the formula: 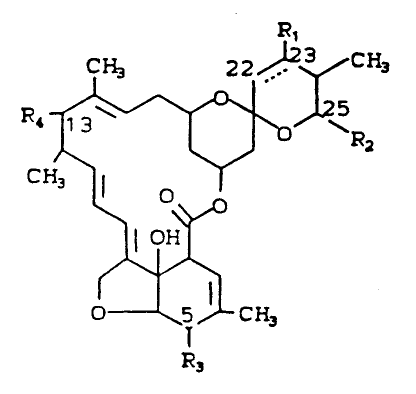 wherein the broken line indicates a single or a double bond at the 22,23-positions;
R₁ is hydrogen or hydroxy provided that R₁ is present only when the broken line indicates a single bond;
R₂ is alkyl of from 1 to 6 carbon atoms or alkenyl of from 3 to 6 carbon atoms or cycloalkyl of from 3 to 8 carbon atoms;
R₃ is hydroxy, methoxy or = NOR₅ where R₅ is hydrogen or loweralkyl; and
R₄ is hydrogen, hydroxy or 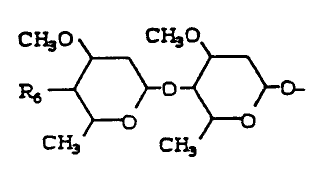
where R₆ is hydroxy, amino, mono- or di-loweralkyl amino or loweralkanoylamino.

4. The composition of Claim 2 wherein the avermectin compound has the formula: 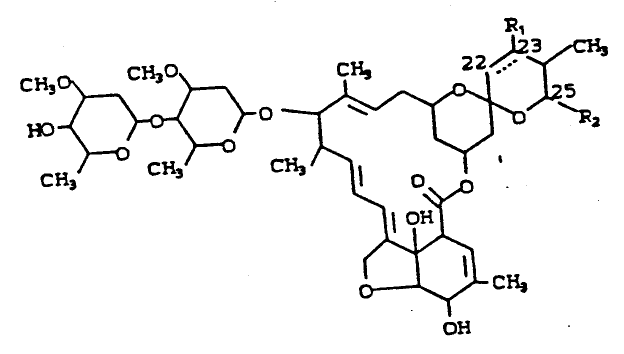 wherein for abamectin the broken line represents double bond and R₁ is not present and for ivermectin the double bond represents a single bond and R₁ is hydrogen; and
R₂ is isopropyl or sec-butyl.

5. The composition of Claim 3 wherein the avermectin compound has the formula: 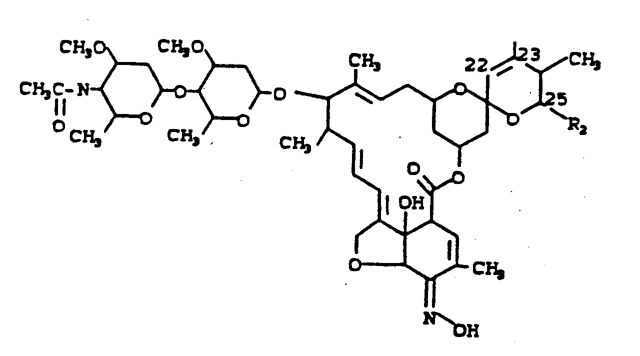 where R₂ is sec-butyl or isopropyl.

6. The composition of Claim 4 where the avermectin compound is ivermectin.

7. The formulation of Claim 1 which contains from about 5 to 50 mg of the avermectin compound per ml of formulation.

8. The process of Claim 1 where the hydrophobic carrier is glyceryl triacetate, distilled acetylated monoglycerides, miglyol™ 812 (caprylic/capric triglyceride), safflower seed oil or mixtures thereof.

9. A process for the preparation of the composition of Claim 1 which comprises dissolving the avermectin compound in the pharmaceutically acceptable hydrophobic carrier; dissolving the optional antioxidant in the resulting solution; heating the solution to about 50°C; adding the hydrogenated castor oil; mixing the resultant solution in a high speed mixer at about 4000 rpm for about 25 minutes.

10. The use of a composition of claim 1 for the manufacture of a medicament for the treatment of internal and external parasites of animals.

## Patentansprüche

1. Injizierbare Zusammensetzung, die aus 0,5 bis 10% Gew./Vol. einer Avermectin-Verbindung in einem pharmazeutisch annehmbaren hydrophoben Träger und 0,5 bis 3% Gew./Vol. eines hydrierten Castoröls besteht.

2. Zusammensetzung nach Anspruch 1, in welcher die Avermectin-Verbindung zu 2,5 bis 5% Gew./Vol. der Formulierung vorliegt.

3. Zusammensetzung nach Anspruch 1, in welcher die Avermectin-Verbindung die Formel: 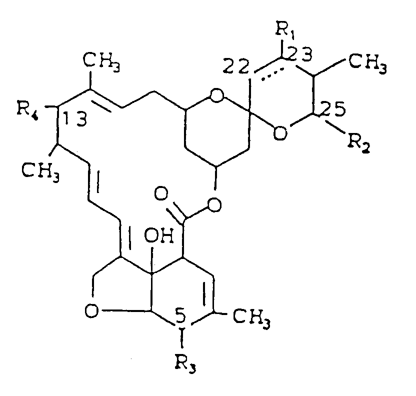 aufweist, in der die gestrichelte Linie eine Einfach- oder Doppelbindung an den 22,23-Positionen anzeigt;
R₁ Wasserstoff oder Hydroxy ist, vorausgesetzt, daß R₁ nur vorhanden ist, wenn die gestrichelte Linie eine Einfachbindung anzeigt;
R₂ Alkyl mit 1 bis 6 Kohlenstoffatomen oder Alkenyl mit 3 bis 6 Kohlenstoffatomen oder Cycloalkyl mit 3 bis 8 Kohlenstoffatomen ist;
R₃ Hydroxy, Methoxy oder = NOR₅ ist, worin R₅ Wasserstoff oder Niederalkyl ist; und
R₄ Wasserstoff, Hydroxy oder 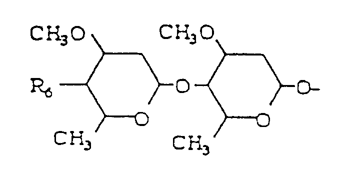
ist, worin R₆ Hydroxy, Amino, Mono- oder Diniederalkylamino oder Niederalkanoylamino ist.

4. Zusammensetzung nach Anspruch 2, in welcher die Avermectin-Verbindung die Formel: 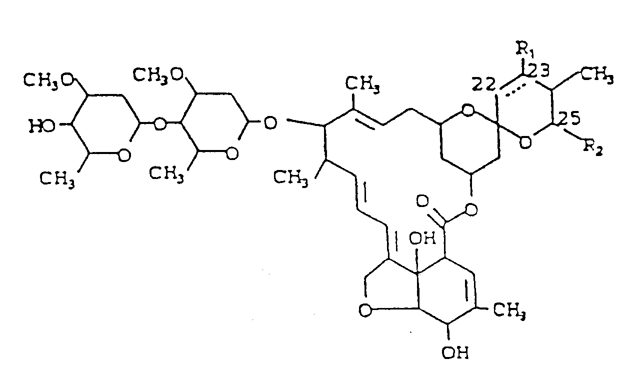 aufweist, in der bei Abamectin die gestrichelte Linie eine Doppelbindung darstellt und R₁ nicht anwesend ist und bei Ivermectin die gestrichelte Linie eine Einfachbindung darstellt und R₁ Wasserstoff ist; und
R₂ Isopropyl oder sek.-Butyl ist.

5. Zusammensetzung nach Anspruch 3, in der die Avermectin-Verbindung die Formel: 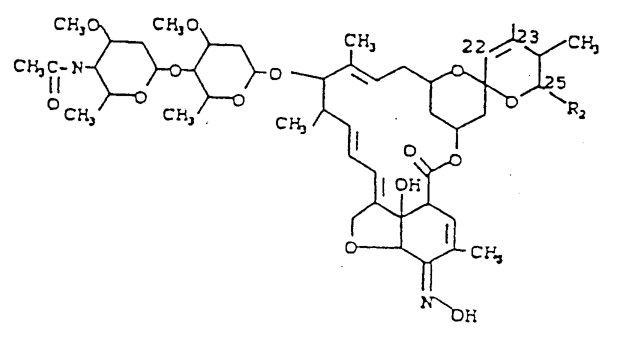 aufweist, in der R₂ sek.-Butyl oder Isopropyl ist.

6. Zusammensetzung nach Anspruch 4, in welcher die Avermectin-Verbindung Ivermectin ist.

7. Zusammensetzung nach Anspruch 1, welche etwa 5 bis 50 mg der Avermectin-Verbindung pro ml Formulierung enthält.

8. Zusammensetzung nach Anspruch 1, in welcher es sich bei dem hydrophoben Träger um Glyceryltriacetat, destillierte acetylierte Monoglyceride, Miglyol™ 812 (Caprylyl/Caprinyltriglycerid), Saflorol oder Mischungen derselben handelt.

9. Verfahren zur Herstellung der Zusammensetzung von Anspruch 1, welches das Lösen der Avermectin-Verbindung in dem pharmazeutisch annehmbaren hydrophoben Träger; das Lösen des fakultativen Antioxidans in der resultierenden Lösung; das Erwärmen der Lösung auf etwa 50°C; die Zugabe des hydrierten Castoröls; das Mischen der resultierenden Lösung in einem Hochgeschwindigkeitsmischer bei etwa 4000 U/min während etwa 25 Minuten umfaßt.

10. Verwendung einer Zusammensetzung von Anspruch 1 für die Herstellung eines Medikaments für die Behandlung von inneren und äußeren Parasiten von Tieren.

## Revendications

1. Composition injectable constituée de 0,5 à 10% en poids/volume d'un composé avermectine dans un véhicule hydrophobe pharmaceutiquement acceptable et de 0,5 à 3% en poids/volume d'une huile de ricin hydrogénée.

2. Composition selon la revendication 1, dans laquelle le composé avermectine est présent à raison de 2,5% à 5% en poids/volume de la formulation.

3. Composition selon la revendication 1, dans laquelle le composé avermectine répond à la formule: 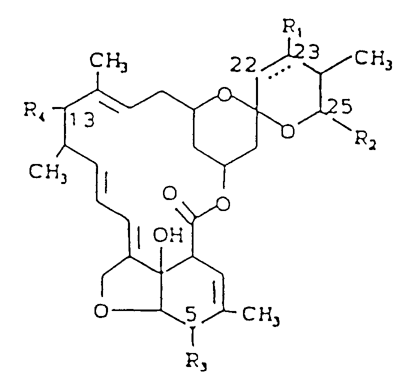 dans laquelle le trait pointillé représente une simple ou une double liaison en position 22,23;
R₁ est un atome d'hydrogène ou un groupe hydroxy à condition que R₁ ne soit présent que lorsque le trait pointillé représente une simple liaison;
R₂ est un groupe alkyle de 1 à 6 atomes de carbone ou alcényle de 3 à 6 atomes de carbone ou cycloalkyle de 3 à 8 atomes de carbone;
R₃ est un groupe hydroxy, méthoxy ou =NOR₅, où R₅ est un atome d'hydrogène ou un groupe alkyle inférieur; et
R₄ est un atome d'hydrogène ou un groupe hydroxy ou 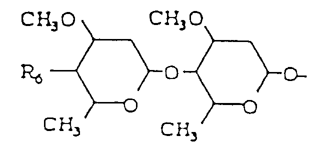
où R₆ est un groupe hydroxy, amino, mono ou dialkyl(inférieur)amino ou alcanoyl(inférieur)amino.

4. Composition selon la revendication 2, dans laquelle le composé avermectine répond à la formule: 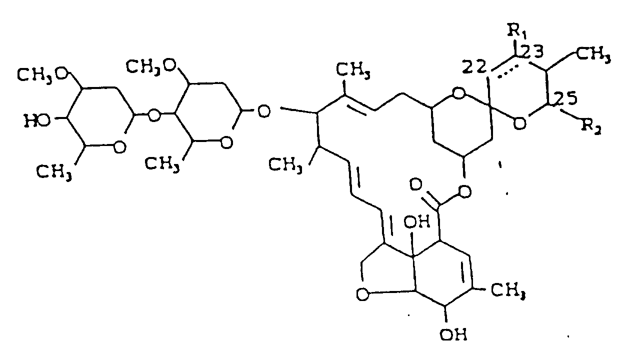 dans laquelle, pour l'abamectine, le trait pointillé représente une double liaison et R₁ n'est pas présent et, pour l'ivermectine, le trait pointillé représente une simple liaison et R₁ est un atome d'hydrogène; et
R₂ est un groupe isopropyle ou sec-butyle.

5. Composition selon la revendication 3, dans laquelle le composé avermectine répond à la formule: 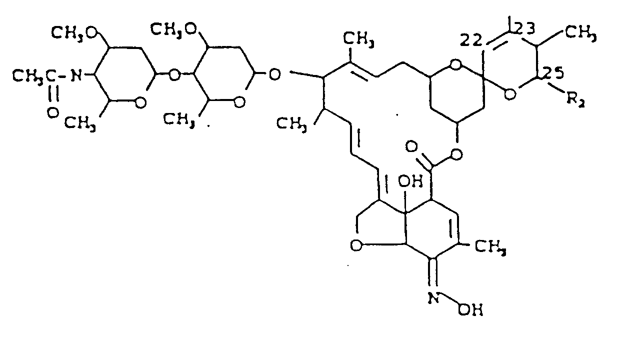 dans laquelle R₂ est un groupe sec-butyle ou isopropyle.

6. Composition selon la revendication 4, dans laquelle le composé avermectine est l'ivermectine.

7. Formulation selon la revendication 1, qui contient d'environ 5 à 50 mg du composé avermectine par ml de formulation.

8. Procédé selon la revendication 1, dans lequel le véhicule hydrophobe est le triacétate de glycéryle, les monoglycérides acétylés distillés, le Miglyol™ 812 (triglycéride caprylique/caprique) l'huile de graine de carthame ou leurs mélanges.

9. Procédé de préparation de la composition selon la revendication 1, qui comprend la dissolution du composé avermectine dans le véhicule hydrophobe pharmaceutiquement acceptable; la dissolution de l'antioxydant facultatif dans la solution résultante; le chauffage de la solution à environ 50°C; l'addition de l'huile de ricin hydrogénée; le mélange de la solution résultante dans un mélangeur à grande vitesse à environ 4000 tours/min. pendant environ 25 minutes.

10. Utilisation d'une composition selon la revendication 1 pour la fabrication d'un médicament pour le traitement de parasites internes et externes chez les animaux.
